# EUROPÄISCHE PATENTSCHRIFT

(11) **EP 0 641 195 B1**
(45) Veröffentlichungstag und Bekanntmachung des Hinweises auf die Patenterteilung: **10.04.1996**
(21) Anmeldenummer: 93912763.5
(22) Anmeldetag: 21.05.1993
(51) Int. Cl.: A61K 9/20, A61K 9/16

(54) **VERFAHREN ZUR HERSTELLUNG RETARDIERTER ARZNEIMITTELZUBEREITUNGEN**
PROCESS FOR PREPARING DELAYED-ACTION MEDICINAL COMPOSITIONS
PROCEDE DE PREPARATION DE COMPOSITIONS MEDICAMENTEUSES A EFFET RETARDE

(30) Priorität: 22.05.1992 DE 4216948
(43) Veröffentlichungstag der Anmeldung: 08.03.1995
(73) Patentinhaber: GÖDECKE AKTIENGESELLSCHAFT, D-10587 Berlin (DE)
(72) Erfinder: RAUCHFUSS, Roland, D-7800 Freiburg (DE)
(74) Vertreter: Mansmann, Ivo
(86) Internationale Anmeldenummer: EP9301289
(87) Internationale Veröffentlichungsnummer: WO9324110

(56) Entgegenhaltungen:
- EP-A- 0 204 596

## Beschreibung

Aus der EP-PS 0 043 254 (B1) ist ein Verfahren zur Herstellung von Arzneimittelzubereitungen mit verzögerter Wirkstoffabgabe bekannt, das auf einem selektiven Aufschmelzprozess mindestens zweier lipider oder lipoider Komponenten beruht, die eine retardierende Wirkung für mit diesen Komponenten vermischte Arzneimittelwirkstoffe haben. Das Verfahren ist dadurch gekennzeichnet, daß man
(a) den Wirkstoff fein verteilt;
(b) den Wirkstoff in fein verteilter Form sowohl mit einer fein verteilten hochschmelzenden lipiden oder lipoiden Komponente als auch mit einer fein verteilten niedrigschmelzenden lipiden oder lipoiden Komponente vermischt, wobei das Gewichtsverhältnis der beiden lipiden oder lipoiden Komponenten im Bereich von 1:5 bis 5:1 liegt;
(c) das resultierende Gemisch aus Wirkstoff und lipiden oder lipoiden Komponenten auf eine Temperatur bringt, die oberhalb des Schmelzpunktes der niedrigschmelzenden Komponente jedoch unterhalb des Schmelzpunkts der hochschmelzenden Komponente liegt, wobei der Wirkstoff und die hochschmelzende lipide oder lipoide Komponente gleichmässig in der vollständig geschmolzenen niedrigschmelzenden lipiden oder lipoiden Komponente dispergiert werden;
(d) das resultierende Gemisch nach dem Aufschmelzen der niedrigschmelzenden Komponente unter deren Schmelzpunkt abkühlen läßt; und
(e) das resultierende Gemisch während des Abkühlens oder danach granuliert, wobei die Angaben "niedrigschmelzend" und "hochschmelzend" in Bezug auf die Relation zueinander verwendet werden, ohne irgendwelche besonderen Schmelzpunkte einzuschließen.

Das genannte Verfahren hat, obwohl es sich technisch bereits gut bewährt hat, auch gewisse Nachteile, die insbesondere einem kontinuierlichen und automatisch gesteuerten Herstellungsprozess im Wege stehen. So ist es bisher nicht möglich, den Aufschmelzprozess kontinuierlich durchzuführen. In der Patentschrift wird zwar unter anderem vorgeschlagen, das Aufschmelzen der niedrigschmelzenden Komponente allein mittels der Friktionswärme eines Extruders zu erzeugen und auf diese Weise auf ein separates Aufheizen der Mischung zu verzichten. Neuerdings durchgeführte Versuche haben jedoch ergeben, daß die Friktionswärme eines Extrusionsverfahrens nicht ausreicht, die niedrigerschmelzende Komponente vollständig aufzuschmelzen. Das erhaltene Extrudat ist deshalb inhomogen und als Grundlage für die Granulierung oder sonstige Weiterverarbeitung zu einem Arzneimittel nicht brauchbar. Bisher ist es folglich nicht möglich, das teilaufgeschmolzene Produkt homogen zu extrudieren. Versucht man nun, die Friktionswärme durch eine Steigerung der Umdrehungszahl der Schnecke zu erhöhen, so tritt, ohne daß die Arbeitstemperatur wesentlich steigt, überraschend eine Entmischung ein, und der Extruder wird durch extremen Druckanstieg vor der Düsenplatte manchmal bis zur Materialzerstörung (Schneckenbruch) beansprucht, ohne daß der gewünschte Effekt erzielt werden könnte. Demgemäß muß gemäß EP-PS 0 043 254 nach wie vor jeder Ansatz für sich in entsprechend dimensionierten Gefäßen gemischt, aufgeheizt und innerhalb des vorgesehenen Zeitschemas wieder abgekühlt werden. Das ist nicht nur zeitaufwendig, sondern zwangsweise auch mit viel Leerlauf für die Reinigung und Wiederbeschickung zwischen den eigentlichen Herstellungsansätzen verbunden. Nach dem bisherigen Herstellungsverfahren in einem 114 kg fassenden Planetenmischer betrug zum Beispiel allein die Aufheizzeit bis zum Schmelzbereich von 58 bis 60 °C eine Stunde. Darüber hinaus ist der Substanzverlust, d. h. der Verlust an Wirk- und Hilfsstoffen, die an den Gefäßwänden hängenbleiben und beim Reinigungsprozeß in der Regel verlorengehen, nicht zu unterschätzen. Ein weiterer Nachteil des Verfahrens ist, daß nach Beendigung des Schmelzprozesses das fertige Gemisch in großen Klumpen oder als Schmelzkuchen anfällt, die zunächst mit Handschaufeln aus dem Schmelzgefäß entfernt und dann zerkleinert werden müssen, bevor man das fertig retardierte Gemisch in einen Granulator überführen kann.

Aber auch Versuche, einen Extruder mit zusätzlicher Heizung einzusetzen, sind anfänglich völlig gescheitert. Es war nämlich zunächst auch unter Wärmezufuhr nicht möglich, ein Extrudat herzustellen. Ausgehend von der naheliegenden Annahme, daß die sehr kurze Verweilzeit von 2 bis 5 Minuten der Mischung im Extruder eine hohe Schmelztemperatur erfordert, wurde als Aufschmelztemperatur ein nur geringfügig unter dem Schmelzpunkt der hochschmelzenden Komponente liegender Temperaturbereich gewählt. Die niedrigschmelzende Komponente wurde dabei erwartungsgemäß aufgeschmolzen, aber es trat gleichzeitig ein unerwarteter Abquetscheffekt auf, der dazu führte, daß die niedrigschmelzende Komponente vom Rest der Mischung abgetrennt und in geschmolzener Form durch die Düsenplatte gepreßt wurde. Die im Extruder verbliebene Mischung wurde somit vom "Schmiermittel" getrennt und verfestigte sich. Dabei erhöhte sich der Friktionswiderstand so, daß der Extruder angehalten wurde. Versuche, dieses Problem durch eine Variation der Umdrehungszahl der Schnecke oder eine Erniedrigung des Durchmessers der Düsen zu meistern, verliefen ergebnislos. Auf eine Erniedrigung der Temperatur wurde verzichtet, da durch diese Maßnahme nur eine weitere Verschlechterung des Ergebnisses erwartet werden mußte. Bei dieser Überlegung wurde insbesondere berücksichtigt, daß eine niedrige Aufschmelztemperatur die Verweildauer der Mischung im Extruder zwangsläufig unerwünscht verlängert, dabei aber den schädlichen Abquetscheffekt schon deshalb nicht günstig beeinflussen kann, weil beim Erreichen der Düsenplatte ohnehin die gesamte niedrigschmelzende Komponente im geschmolzenen Zustand vorliegen muß und mithin unter dem im Extruder herrschenden Druck von 200 bis 600 kPa (N/m²) ebenso abgequetscht wird wie bei höheren Temperaturen. Ein irgendwie gearteter Temperatureinfluß auf den Abquetscheffekt wurde nicht erwartet. Im übrigen war zu erwarten, daß langsames Aufheizen und mithin niedrigere Temperaturen in der Aufheizphase nicht nur den Durchsatz verringern, sondern auch einen sehr langen Kompressionsweg und damit aufwendige Apparaturen erfordern würden.

Aufgabe der vorliegenden Erfindung ist es, die obengenannten Nachteile zu überwinden und einen vollautomatisch und kontinuierlich arbeitenden Schmelzprozess nach EP - PS 0 043 254 bereitzustellen.

Überraschenderweise hat sich nun herausgestellt, daß der schädliche Abquetscheffekt wider erwarten und bisher nicht erklärlich bei einer einfachen Absenkung der Arbeitstemperaur in den untersten möglichen Bereich vollkommen verschwindet und daS sich folglich die aus der EP - PS 0 043 254 bekannten und dort vorgeschlagenen Mischungen trotz der vielen vergeblichen Versuche ohne besondere Veränderung der Zusammensetzung, insbesondere ohne irgendwelche Zuschläge zur Modifizierung der Friktion, zu hervorragend weiterzuverarbeitenden Extrudaten verpressen lassen, wenn man die gut zerkleinerte und vorgemischte pulverförmige Masse bei einer Temperatur, die höchstens 4°C über der Schmelztemperatur der niedrigerschmelzenden lipiden oder lipoiden Komponente liegt bei einem Druck von 200 bis 600 kPa (N/m²) einem Extrusionsprozess unterwirft und die gemäß EP - PS 0 043 254 teilaufgeschmolzene und gut vermischte Masse durch eine Düsenplatte mit einem Düsendurchmesser von 1,2 bis 4 mm extrudiert.

Erfindungsgemäß soll die Temperatur im Extruder nicht mehr als 1 bis 4, bevorzugt 1 bis 3, am günstigsten jedoch nur 1 bis 2 °C über der Schmelztemperatur der niedrigschmelzenden Komponente liegen. Die Temperatur des Heizmantels muß also entsprechend so eingestellt werden, daß diese Temperaturbereiche in der zu verarbeitenden Mischung über die gesamte Schneckenlänge eingehalten werden. Lediglich kurz vor dem Passieren der Düsen kann die Temperatur in den Bereich der Erstarrungstemperatur der niedrig schmelzenden Komponente abgesenkt werden, wenn durch ausreichende Fördergeschwindigkeit dafür gesorgt wird, daß diese Komponente erst nach dem Passieren der Düsenplatte erstarren kann.

Aber auch wenn die Temperatur im oben angegebenen optimalen Bereich liegt, erhält man ein brauchbares, d. h. granulierbares und in ein Arzneimittel überführbares Produkt, erst dann, wenn man gleichzeitig den Durchmesser der Düsen auf eine an das Verfahren angepaßte Größe einstellt. Beträgt der Düsendurchmesser weniger als 1 mm, so verstopft die Düsenplatte und der Extruder wird durch die zunehmende Friktion angehalten, was zu erheblichen Schäden an der Maschine führen kann. Wird andererseits der Durchmesser zu groß, d.h. größer als 4 mm bemessen, so erhält man ein zwar brauchbar aussehendes Produkt, das aber in Wirklichkeit nur auf der Oberfläche angeschmolzen ist. Man erhält sozusagen einen Schlauch mit einer aufgeschmolzenen Wandung und pulverförmiger Füllung. Es kam also auch hier darauf an, den richtigen Durchmesserbereich aufzufinden. Dieser liegt bei 1,2 bis 4, bevorzugt 1,3 bis 3, und ganz besonders bevorzugt bei 1,5 bis 2 mm.

Die Umdrehungszahl der Schnecke(n) wird entsprechend dem verwendeten Extruder und dem zu verarbeitenden Gemisch so eingestellt, daß der angestrebte Verfahrensdruck von 200 bis 500 kPa (N/m²) erzielt wird. Eine typischer Umdrehungsbereich für einen Extruder mit einer Schneckenlänge von 1200 mm liegt bei einem Schmelzendruck von 200 bis 600 kPa (N/m²) zwischen 50 und 200 UpM.

Das erfindungsgemäße Verfahren hat gegenüber der aus dem obengenannten Stand der Technik bekannten Verfahren erhebliche Vorteile. Durch die kontinuierliche Herstellungsweise kann die Fertigungszeit für eine Einheitsmenge erheblich reduziert werden. Beispielsweise benötigte man bisher für die Herstellung von 450 kg Granulat 16 Mannstunden. Dieselbe Menge wird nach dem erfindungsgemäßen Verfahren in nur 4 Mannstunden hergestellt. Die reine Herstellungszeit wird dabei um ca. 50% reduziert. Der Extruder benötigt wenig Platz und arbeitet sehr ökonomisch. Beispielsweise können mit einem Extruder einer Schneckenlänge von nur 1400 mm stündlich 110-130 kg Extrudat hergestellt werden.

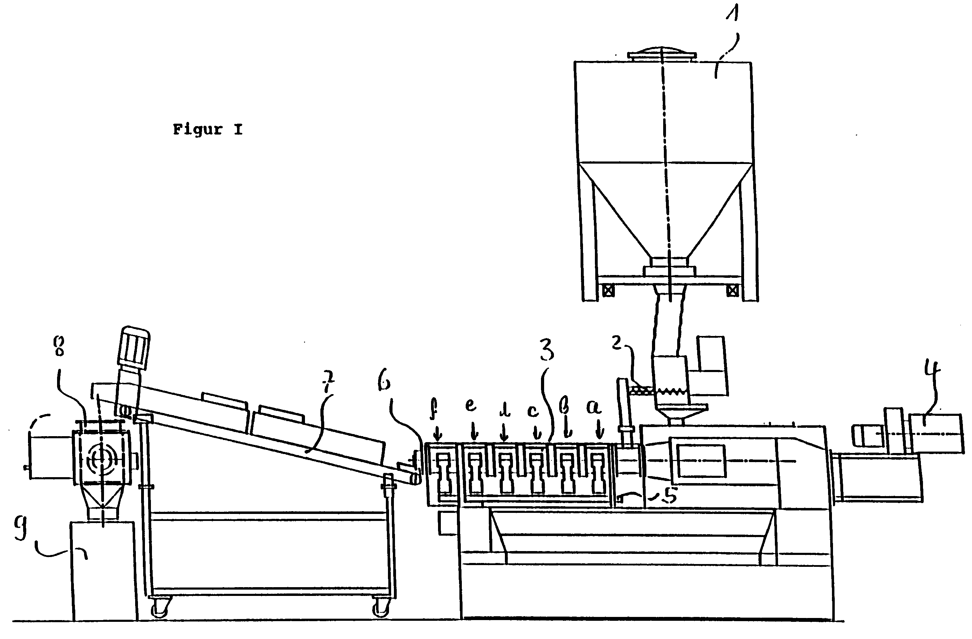

Das erfindungsgemäße Verfahren wird im folgenden anhand der Figur I näher beschrieben:
Aus einem Vorratsbehälter (1) wird ein vorbereitetes fein verteiltes Gemisch bestehend aus:
37,5 kg Rizinusöl hydriert, Schmelztemperatur: 80-85°C
60,0 kg Stearinsäure plv., Schmelztemperatur: 55-56°C
90 kg Diltiazemhydrochlorid
255,5 kg Lactose K
1,5 kg Magnesiumstearat
1,75 kg Carboxymethylcellulose
über eine Dosierschnecke (2) dem eigentlichen Extruder (3) zugeleitet. Der Extruder (3) ist in mehrere getrennt temperierbare Schüsse (a-f) eingeteilt und wird über den steuerbaren Motor (4) angetrieben. Die Mischung wird bei einer Umdrehungszahl von 170-180 UpM in den durch den heizbaren Mantel (5) vorgeheizten Extruder (3) gefördert.

Der Schneckendurchmesser beträgt 50 mm. Die Manteltemperatur beträgt in allen Schüssen 58-60°C; das entspricht einer Arbeitstemperatur von 58-60°C. Nach einer durchschnittlichen Verweildauer von 2-4 Minuten wird das teilaufgeschmolzene Produkt durch die Düsenplatte (6) extrudiert. Die Düsenplatte (6) enthält 20 Düsenöffnungen mit einem inneren Durchmesser von 1,5-3 mm. Die Apparatur fördert unter den beschriebenen Bedingungen 110-120 kg Extrudat in der Stunde. Das Extrudat wird in Form feiner gleichmäßiger Stränge auf dem langsam laufenden Förderband weitgehend auf Raumtemperatur abgekühlt und schließlich dem Granulator (8) zugeführt. das fertige Granulat gelangt schließlich in den Auffangbehälter (9).

Die Schüsse a bis f werden im einfachsten Fall gleichmäßig temperiert, so daß beim Durchlaufen des Extrusionsprozesses über die ganze Länge der Schnecke (1400 mm) dieselbe Arbeitstemperatur herrscht. Es ist jedoch auch möglich, anfänglich bei etwas höherer Temperatur zu arbeiten und diese graduell abzusenken, bis etwa im letzten Schuß (f) die Arbeitstemperatur nahezu auf die Erstarrungstemperatur der niedrigerschmelzenden Komponente abgesenkt ist. Dadurch wird die Abkühlungsphase etwas verkürzt, ohne daß es zu einem Verstopfen der Düsen kommt.

Im übrigen können die in der EP-PS 0 043 254 beschriebenen Gemische ohne Veränderung eingesetzt und verarbeitet werden.

Ein Ansatz für die Herstellung von 60 mg Diltiazemzübereitungen enthält z.B.
96 kg Diltiazemhydrochlorid
272 kg Lactose K
40 kg Rizinusöl hydriert
4,8 kg Carboxymethylcellulose
64 kg Stearinsäure
1,6 kg Magnesiumstearat
Ein Ansatz für die Herstellung von 120 mg Diltiazem-Zubereitungen enthält z.B.
120 kg Diltiazemhydrochlorid
215,3 kg Lactose K
64 kg Stearinsäure NF
40 kg Rizinusöl hydriert
2,25 kg Carboxymethylcellulose
2,25 kg Magnesiumstearat
Ein Ansatz für die Herstellung von 180 mg Diltiazem-Zübereitungen enthält z.B.
180 kg Diltiazemhydrochlorid
144,124 kg Lactose K
48 kg Rizinusöl hydriert
68,2 kg Stearinsäure NF
1,124 kg Hydroxyethylcellulose
2,3 kg Magnesiumstearat
Ein Ansatz für die Herstellung von 240 mg Diltiazem-Zubereitungen enthält z.B.
13,5 kg Lactose K
24 kg Diltiazemhydrochlorid
12 kg Rizinusöl hydriert
10 kg Stearinsäure
0,175 kg Hydroxyethylcellulose
0,4 kg Magnesiumstearat
Ein Ansatz für die Herstellung von Norfenefrin-Zubereitungen enthält z.B.
58,5 kg Norfenefrinhydrochlorid
152,1 kg Lactose K
5,85 kg Titandioxid
29,25 kg Rizinusöl hydriert
43,876 kg Stearinsäure NF
2,924 kg Carboxymethylcellulose
Ein weiterer Ansatz für die Herstellung von Norfenefrin-Zubereitungen enthält z.B.
30 kg Norfenefrinhydrochlorid
166 kg Lactose K
6 kg Titandioxid
30 kg Rizinusöl hydriert
44 kg Stearinsäure NF
4 kg Carboxymethylcellulose

## Patentansprüche

1. Verfahren zur Herstellung einer Arzneimittelzübereitung mit verzögerter Wirkstoffabgabe, bei welchem man
(a) den Wirkstoff fein verteilt;
(b) den Wirkstoff in fein verteilter Form sowohl mit einer fein verteilten hochschmelzenden lipiden oder lipoiden Komponente als auch mit einer fein verteilten niedrigschmelzenden lipiden oder lipoiden Komponente vermischt, wobei das Gewichtsverhältnis der beiden lipiden oder lipoiden Komponenten im Bereich von 1:5 bis 5:1 liegt;
(c) das resultierende Gemisch aus Wirkstoff und lipiden oder lipoiden Komponenten auf eine Temperatur bringt, die oberhalb des Schmelzpunktes der niedrigschmelzenden Komponente jedoch unterhalb des Schmelzpunkts der hochschmelzenden Komponente liegt, wobei der Wirkstoff und die hochschmelzende lipide oder lipoide Komponente gleichmässig in der vollständig geschmolzenen niedrigschmelzenden lipiden oder lipoiden Komponente dispergiert werden;
(d) das resultierende Gemisch nach dem Aufschmelzen der niedrigschmelzenden Komponente unter deren Schmelzpunkt abkühlen läßt; und
(e) das resultierende Gemisch während des Abkühlens oder danach granuliert, wobei die Angaben "niedrigschmelzend" und "hochschmelzend" in Bezug auf die Relation zueinander verwendet werden, ohne irgendwelche besonderen Schmelzpunkte einzuschließen,
dadurch gekennzeichnet,
daß man die gut zerkleinerte und vorgemischte pulverförmige Masse gemäß (b) mittels einer Extruderschnecke in einen vortemperierten Extruder einführt und so gemäß (c) auf eine Temperatur bringt, die höchstens 4°C über der Schmelztemperatur der niedrigschmelzenden lipiden oder lipoiden Komponente liegt, das so erwärmte Gemisch bei einem Druck von 200 bis 600 kPa (N/m²) einem Extrusionsprozess unterwirft und es auf diese Weise dispergiert, wobei die teilaufgeschmolzene und gut vermischte Masse durch eine Düsenplatte mit einem Düsendurchmesser von 1,2 bis 4 mm extrudiert und anschließend gemäß (d) abgekühlt und gewünschtenfalls anschließend granuliert wird.

2. Verfahren nach Anspruch 1, dadurch gekennzeichnet, daß die Temperatur gemäß (c) 1 bis 4 °C über der Schmelztemperatur der niedrigschmelzenden lipiden oder lipoiden Komponente liegt.

3. Verfahren nach Anspruch 1 und 2, dadurch gekennzeichnet, daS die Temperatur gemäß (c) 1 bis 3 °C über der Schmelztemperatur der niedrigschmelzenden lipiden oder lipoiden Komponente liegt.

4. Verfahren nach Anspruch 1 bis 3, dadurch gekennzeichnet, daß die Temperatur gemäß (c) 1 bis 2 °C über der Schmelztemperatur der niedrigschmelzenden lipiden oder lipoiden Komponente liegt.

5. Verfahren gemäß Anspruch 1 bis 4, dadurch gekennzeichnet, daß der Düsendurchmesser in der Düsenplatte jeweils 1,2 bis 4 mm beträgt.

6. Verfahren gemäß Anspruch 1 bis 5, dadurch gekennzeichnet, daß der Düsendurchmesser in der Düsenplatte jeweils 1,3 bis 3 mm beträgt.

7. Verfahren gemäß Anspruch 1 bis 6, dadurch gekennzeichnet, daß der Düsendurchmesser in der Düsenplatte jeweils 1,5 bis 2 mm beträgt.

8. Verfahren nach Anspruch 1 bis 7, dadurch gekennzeichnet, daß der Schmelzdruck des Verfahrens bei 200 bis 600 kPa (N/m²) gehalten wird.

## Claims

1. Process for producing a medicine with delayed action of an effective constituent, in which
(a) an effective constituent is finely distributed;
(b) an effective constituent in finely distributed form is mixed both with a finely distributed, high-melting lipid or lipoid constituent and with a finely distributed low-melting lipid or lipoid constituent, whereby a weight ratio of twolipid or lipoid constituents lies in a range of from 1:5 to 5:1;
c) a resulting mixture of an effective constituent and lipid or lipoid constituents is brought to a temperature which is over a melting-point of a low-melting constituent, yet below a melting-point of a high-melting constituent, whereby an effective constituent and a high-melting lipid or lipoid constituent are uniformly dispersed in a fully melted low-melting lipid or lipoid constituent;
d) a resulting mixture is cooled, after melting of a low-melting constituent, to below its melting-point; and
e) a resulting mixture is granulated during or after cooling, whereby terms 'low-melting' and 'high-melting' are used in relation to each other without including any particular melting-point,
wherein
a previously reduced and well mixed powder material in accordance with b) is introduced into an extruder, which is at a previously regulated temperature, by means of an extrusion screw and is thereby brought to a temperature in accordance with c) which is, at most, 4° C over a melting temperature of a low-melting lipid or lipoid constituent, and a mixture heated in this way is subjected to an extrusion process at a pressure of 200 to 600 kPa (N/m²), whereby a partly melted and thoroughly mixed material is extruded through a nozzle plate with a nozzle-diameter of 1.2 to 4 mm and is then cooled and, where applicable, granulated, in accordance with d).

2. Process in accordance with claim 1, wherein a temperature is from 1 to 4° C over a melting-temperature of a low-melting lipid or lipoid constituent, in accordance with c).

3. Process in accordance with claim 1 and claim 2, wherein a temperature in accordance with c) is 1 to 3° C above a melting-temperature of a low-melting lipid or lipoid constituent.

4. Process in accordance with claims 1 to 3, wherein a temperature, in accordance with c), is 1 to 2° C above a melting-temperature of a low-melting lipid or lipoid constituent.

5. Process in accordance with claims 1 to 4, wherein a nozzle-diameter in a nozzle plate is in each case 1.2 to 4 mm.

6. Process in accordance with claims 1 to 5, wherein a nozzle-diameter in a nozzle plate is in each case 1.3 to 3 mm.

7. Process in accordance with claims 1 to 6, wherein a nozzle diameter in a nozzle plate is, in each case, 1.5 to 2 mm.

8. Process in accordance with claims 1 to 7, wherein a melting-pressure of a process concerned is kept at 200 to 600 kPa (N/m²).

## Revendications

1. Procédé de préparation d'une composition pharmaceutique à libération retardée du principe actif, dans lequel:
(a) on divise finement le principe actif;
(b) on mélange le principe actif finement divisé avec un composant lipidique ou lipoïdique à haut point de tusion finement divisé également, ainsi qu'avec un composant lipidique ou lipoïdique à bas point de fusion finement divisé, le rapport en poids des deux composants lipidiques ou lipoïdiques allant de 1/5 à 5/1 ;
(c) on porte le mélange résultant de principe actif et de composants lipidiques ou lipoïdiques à une température supérieure au point de fusion des composants à de plus bas point de fusion mais inférieure au point de fusion des composants à point de fusion plus élevés, de sorte que le principe actif et le composant lipidique ou lipoïdique à point de fusion plus élevé soient dispersés uniformément dis les composants lipidiques ou lipoïdiques à plus bas point de fusion complètement fondu;
(d) on laisse refroidir le mélange résultant après fusion du composant à bas point de fusion à une température inférieure à son point de fusion ; et
(e) on granule le mélange résultant pendant ou après le refroidissement, les expressions "à bas point de fusion" et "à haut point de fusion" étant considérés l'un par rapport à l'autre, sans tenir compte de quelconques points de fusion particuliers,
caractérisé en ce que:
on introduit la masse pulvérulente finement divisé et bien mélangée de l'étape (b) dans une extrudeuse préchauffée au moyen d'une vis d'extrusion et, conformément à l'étape (c), on porte le mélange à une température qui est au maximum de 4°C supérieure à la température de fusion du composant lipidique ou lipoïdique de plus bas point de fusion, on soumet le mélange ainsi chauffé à une opération d'extrusion sous une pression de 200 à 600 kPa (N/m²) et procéder ainsi à sa dispersion, on extrude la masse partiellement fondue et bien mélangée par une filière ayant un diamètre de 1,2 à 4 mm, puis on la refroidit conformément à l'étape (d), puis, si on le souhaite, on la granule.

2. Procédé selon la revendication 1, caractérisé en ce que la température de l'étape (c) dépasse de 1 à 4°C le point de fusion des composants lipidiques ou lipoïdiques de plus bas point de fusion.

3. Procédé selon la revendication 1 ou 2, caractérisé en ce que la température de l'étape (c) dépasse de 1 à 3°C le point de fusion des composants lipidiques ou lipoïdiques de plus bas point de fusion.

4. Procédé selon les revendications 1 à 3, caractérisé en ce que la température de l'étape (c) dépasse de 1 à 2°C le point de fusion des composants lipidiques ou lipoïdiques des plus bas point de fusion.

5. Procédé selon les revendications 1 à 4, caractérisé en ce que le diamètre de la filière est compris entre 1,2 et 4 mm.

6. Procédé selon les revendications 1 à 5, caractérisé en ce que le diamètre de la filière est compris entre 1,3 et 3 mm.

7. Procédé selon les revendications 1 à 6, caractérisé en ce que le diamètre de la filière est compris entre 1,5 et 2 mm.

8. Procédé selon les revendications 1 à 7, caractérisé en ce que la pression de fusion du procédé est maintenue à une valeur de 200 à 600 kPa (N/m²).
